# EUROPEAN PATENT APPLICATION

(11) **EP 1 219 288 A2**
(43) Date of publication of application: **03.07.2002**
(21) Application number: 01310844.4
(22) Date of filing: 21.12.2001
(51) Int. Cl.: A61K 7/48

(54) **Treatment for skin**

(30) Priority: 21.12.2000 US 742622
(71) Applicant: Johnson & Johnson Consumer Companies, Inc., Skillman, NJ 08558-9418 (US)
(72) Inventor: Cole, Curtis, Ringoes, NJ 08551 (US); Ganopolsky, Irina, Lawrenceville, NJ 08648 (US); Lukenbach, Elvin, Flemington, NJ 08822 (US); Skover, Gregory, Princeton, NJ 08540 (US)
(74) Representative: Mercer, Christopher Paul

(57) **Abstract**

The invention relates to a topical composition for the treatment of skin comprising an effective amount of a compound of the formula: 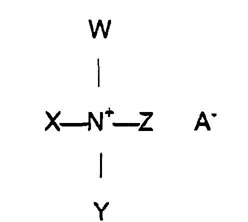 wherein W, X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group, and wherein A is a mixture of anionic counterions derived from at least two pharmaceutically acceptable acids and esters thereof; and a cosmetically acceptable carrier.

## Description

### CROSS REFERENCE TO RELATED APPLICATION

This application claims priority to U.S. Provisional Application Serial No. 60/237,230, filed October 2, 2000, the disclosure of which is hereby incorporated by reference.

### FIELD OF THE INVENTION

This invention relates to compositions and method for improving skin contour by restoring youthful mechanical properties of the skin. More particularly, it relates to compositions containing at least one alkanolamine salts, and their application to mammalian skin to increase the suppleness and compliance of affected skin areas.

### BACKGROUND OF THE INVENTION

Human beings have long sought products that can reverse or diminish the effects of aging without cosmetic surgery. The skin is composed primarily of water and as we age it loses its ability to retain water resulting in a surface that is dry and rough. This is not only due to a decrease in the water-retaining capacity of the stratum corneum, a decrease in barrier function and a decrease in the amount of secretion of sebum but also modulation of matrix molecules in the dermis that support epidermis. Matrix molecules comprised of proteins and polysaccharides become more crosslinked. This results in a decrease of water in the dermal compartment consequently producing a stiff, non-compliant structure. Aging of both skin and other tissues is, in part, the result of constant free radical damage leading to decreased cell function and matrix flexibility. In addition, sunlight exposure intensifies and augments the aging process. Extensive sun exposure results in photodamage and is manifested as lines, mottling, discoloration, precancers and cancers.

In addition to changes on the surface of the skin deeper changes occur in the dermis that effect it's mechanical properties making it more leathery and hardened. Free radical damage induces abnormal interfibrillar crosslink formation. This leads to degradation and atrophy of the matrix. As these crosslinks are formed dermal water is excluded.

Current treatments for these environmental insults include moisturizers, chemical peels, and laser surgery to either hydrate the surface of the skin, enhance epidermal turnover or remove the "hardened" dermal or supportive layers. The more aggressive procedures remove skin tissue and enhance epidermal regeneration and new matrix synthesis thereby restoring some of the original mechanical properties associated with young, undamaged skin.

Japanese Kokai Patent Application No. 495008 discloses an aging inhibitor effect of ethanolamine derivatives which indicates a preventative activity of aging skin effects. According to this application, the age inhibiting compound can be used as a therapeutic to treat aged skin to improve skin elasticity and wrinkles.

U.S. Patent No. 5,554,647 to Perricone discloses a method for percutaneously treating aging skin using ethanolamine ingredients in a dermatologically acceptable carrier. The ethanolamine is selected from the group consisting of dimethylaminoethanol, monoaminoethanol, choline, serine, acetic acid esters of dimethylaminoethanol, acetic acid esters of monoaminoethanol, para-chlorophenylacetic acid esters of dimethylaminoethanol, para-chlorophenylacetic acid esters of monoaminoethanol, and mixtures thereof. These compounds are hypothesized to treat aging skin via a mechanism of neuromuscular stimulation.

Not wishing to be bound to this hypothesis, we have additionally discovered that the superficial biomechanical properties of the skin are affected by application of these same compounds restoring youthful firmness resulting in improved facial contours.

Thus, it is an object of this invention to provide topical compositions that can be used to improve facial contours by modulating the skin's biomechanical properties producing more youthful characteristics.

It is another object of this invention to provide stable topical compositions comprising specific alkanolamine compounds that can be utilized to provide the benefits described above.

### SUMMARY OF INVENTION

It has been discovered that formulations containing alkanolamine salts, significantly increase skin firmness and make skin contours more visible resulting in a more youthful appearance.

Accordingly, the invention relates to a topical composition for the treatment of skin comprising an effective amount of a compound of the formula: 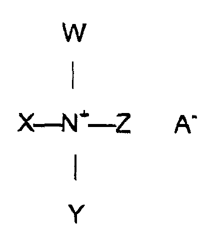 wherein W, X, Y and Z are selected from the group consisting of hydrogen. C₁-C₃ alkyl group, C₂-C₄ alkanol group, wherein at least one of X, Y or Z is a C₂-C₄ alkanol group bearing at least one hydroxyl group and optionally at least one carboxyl group, and wherein A is a mixture of anionic counterions derived from at least two pharmaceutically acceptable acids and esters thereof; and a cosmetically acceptable carrier.

Preferably, the compositions used in the methods according to the invention have a pH ranging from about 4.5 to about 8.5, more preferably, from about 5.5 to about 8.5, more preferably from about 5.5 to about 7.5

The invention also relates to methods for the treatment of aging skin, in particular, for increasing the suppleness and/or compliance of mammalian skin, using a topically-applied composition.

### DETAILED DESCRIPTION OF THE INVENTION

The amount of the alkanolamine salt necessary to reverse or diminish the effects of aging on the skin is not fixed per se, and necessarily is dependent upon the severity and extent of the aged tissue and the concentrations of the ingredients in the formulation put together in association with a dermatologically acceptable carrier.

Generally, even low concentrations of active ingredients in a carrier will be suitable, requiring only that more frequent topical application be resorted to. As a practical matter, however, to avoid the need for repeated application, it is desirable that the topically applied composition be formulated to contain at least about 1% by weight and most preferably at least about 3% by weight, of alkanolamine salt.

The alkanoamine salt can be made by techniques known in the art by reacting the desired alkanolamine with an appropriate acid under conditions sufficient to form the salt. The salts can be formed *in situ* or prior to formulating. Generally, when at least one of W, X, Y, or Z is hydrogen the alkanolamine salt can be prepared *in situ.*

In a preferred embodiment, the alkanoloamine salt is an acid salt of monomethylaminoethanol, dimethylaminoethanolamine, trimethylaminoethanol, isopropanolamine, triethanolamine, isoropanoldimethylamine, ethylethanolamine, 2-butanolamine, choline, serine, and copolymers thereof.

Suitable acids for use in the preparation of the alkanolamine salts according to the invention include any organic acid known to be useful in skin care compositions. In a preferred embodiment, at least one of the acids is an alpha hydroxy acid, such as taught for example in U.S. Patent No. 5,856,357, the disclosure of which is hereby incorporated by reference. Particularly preferred is a mixture of at least two of glycolic acid, malic acid and citric acids. In a most preferred embodiment, the acid is a combination of glycolic acid and either malic or citric acid. In situations where pH stability is a particular concern, e.g., long term storage, a particularly preferred embodiment is when the acid is a mixture of citric and glycolic acid. Preferably, the ratio of malic or citric acid to glycolic acid ranges from about 1:1 to about 1:5, more preferably, from about 1:1 to about 1:3.

Another compound which is advantageously present in the compositions of this invention is tyrosine. Tyrosine may be present in the compositions of this invention in the amount of from about 0.01 to about 5%, more preferably from about 0.04 to about 3% by weight and most preferably about 0.5% by weight, based on the total composition.

Compositions of the invention are applied in admixture with a dermatologically acceptable carrier or vehicle (e.g., as a lotion, cream, ointment, cleanser, or the like). The carriers should be chosen which can solubilize or disperse the ingredients at the concentrations described above. Topical application is facilitated and, in some cases, additional therapeutic effects are provided as might be brought about, e.g., by moisturizing of the affected skin areas. When a carrier is employed, it is necessary that the carrier be inert in the sense of not bringing about a deactivation of the ethanolamine, and in the sense of not bringing about any adverse effect on the skin to which it is applied.

Suitable carriers include water, alcohols, oils and the like, chosen for their ability to dissolve or disperse the active ingredients at concentrations of active ingredients most suitable for use in the therapeutic treatment.

The compositions of this invention should be in the form of topical products that can be applied externally to the skin and can be prepared in accordance with conventional techniques known to those of ordinary skill in the art. The carrier may take a variety of physical forms such as, for example, creams, dressings, gels, lotions, ointments or liquids, including leave-on and rinse-off compositions, as well as incorporated into material carriers such as dry and wet wipes, puffs, hydro-gel matrixes, or adhesive (or non-adhesive) patches by means known in the art. Preferably, the carrier is a gel or moisturizing lotion, a cooling solution or in the form of a dry or wet wipe. One could also utilize this in a convenient spray applicator.

Typical carriers include lotions containing water and/or alcohols and emollients such as hydrocarbon oils and waxes, silicone oils, hyaluronic acid, vegetable, animal or marine fats or oils, glyceride derivatives, fatty acids or fatty acid esters or alcohols or alcohol ethers, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophillic colloids. Such compositions are referred to herein as cosmetically acceptable carriers. Preferably, the carrier should be a gel base formula without lipid materials that would exxacerbate the oiliness of acne prone skin. However, a moisturizer emulsion base may be preferred by individuals that have particularly dry yet skin still suffer from acne lesions.

The topical compositions according to the invention can comprise additional ingredients commonly found in skin care compositions, such as for example, emollients, skin conditioning agents, emulsifying agents, humectants, preservatives, antioxidants, perfumes, chelating agents, etc., provided that they are physically and chemically compatible with the other components of the composition. Noteably useful is the incorporation of vitamin A and vitamin A derivatives, including but not restricted to retinol, retinyl palmitate, retinoic acid, retinal, and retinyl propionate.

Examples of suitable preservatives for use in the compositions of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In a preferred embodiment, the preservative is mixture of from about 0.2 to about 0.5 weight percent methylparaben, from about 0.2 to about 5.0 weight percent propylparaben and from about 0.05 to about 0.10 weight percent butylparaben. A particularly preferred commercially available preservative that may be used in the skin care composition according to this invention is PHENONIP TM which is a practically colorless, viscous, liquid mixture of phenoxyethanol, methylparaben, ethylparaben, propylparaben, and butylparaben available from Nipa Laboratories, Inc., Wilmington, Del.

Preferably, antioxidants should be present in the compositions according to the invention. Suitable antioxidants include butylated hydroxy toluene (BHT), ascorbyl palmitate, butylated hydroanisole (BHA), phenyl-α-naphthylamine, hydroquinone, propyl gallate, nordihydroquiaretic acid, vitamin E or derivatives of vitamin E, vitamin C and derivatives thereof, calcium pantothenic, green tea extracts and mixed polyphenosis, and mixtures thereof of the above. When utilized the antioxidant can be present in an amount ranging from about 0.02 to about 0.5% by weight, more preferably from about 0.002 to about 0.1% by weight of the total composition.

Emollients which can be included in the compositions of the invention function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearate, dimethicone, and combinations thereof. Cetyl alcohol, octyl hydroxystearate, dimethicone, and combinations thereof are preferred. When utilized, the emollient can be present in an amount from about 0.01 to about 5, preferably from about 1 to about 4 percent by weight based on the total composition.

Polyhydric alcohols can be utilized as humectants in the compositions of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 5, preferably from about 1 to about 3 percent by weight, based on the total weight of the composition.

The compositions according to the invention preferably contain an effective stabilizing amount of an emulsifier. Preferably, the emulsifier is present at from about 1.0 to about 10.0, more preferably from about 3.0 to about 6.0, weight percent, based on the total composition. Any emulsifier that is compatible with the components of the composition can be employed. Suitable emulsifiers include stearic acid, cetyl alcohol, stearyl alcohol, steareth 2, steareth 20, Acrylates/C10-30 alkyl Acrylate Crosspolymer. Particularly preferred is PEMULEN TR-1 (CTFA Designation: Acrylates/10-30 Alkyl Acrylate Crosspolymer).

Any fragrance may be added to the compositions of the invention for aesthetic purposes. Suitable fragrances include, but are not limited to, eucalyptus oil, camphor synthetic, peppermint oil, clove oil, lavender, chamomile and the like. When utilized, fragrances are present in an amount from about 0.05 to about 0.5, preferably from about 0.1 to about 0.3 percent by weight, based on the total weight of the composition. In certain aspects of this invention, the compositions should include a chelating agent. Chelating agents which are useful in the compositions of the present invention include ethylenediamine tetra acetic acid (EDTA) and derivatives and salts thereof, dihydroxyethyl glycine, tartaric acid, and mixtures thereof. The chelating agents should be utilized in a stabilizing effective amount and may range from about 0.01 to about 2% based on the weight of the total composition, preferably from about 0.05 to about 1%. Most preferably, the chelating agent should be EDTA.

Generally, the composition is topically applied to the affected skin areas in a predetermined or as-needed regimen to bring about improvement, it generally being the case that gradual improvement is noted with each successive application. Insofar as has been determined based upon clinical studies to date, no adverse side effects are encountered.

While not wishing to be bound to any theory, it is proposed that treatment in accordance with the present invention may induce changes in the skin permitting a repartioning of water in cellular membranes as well as dermal matrix molecules. Rather than water being excluded from the dermis the composition may enhance the inclusion of water from the blood. Enhancing water retention in the dermal tissue increases firmness resulting in more improved facial contours visualized as a more youthful appearance.

The advantages of the invention and specific embodiments of the skin care compositions prepared in accordance with the present invention are illustrated by the following examples. It will be understood, however, that the invention is not confined to the specific limitations set forth in the individual examples, but rather to the scope of the appended claims.

The following materials were used in the Examples that follow:
AMPHISOL A: cetyl palmitate thickener commercially available from Gattefosse.
AMIGEL: sclertoium gum thickener commercially available from Alban Muller
AMIPHISOL A : cetyl phosphate emulsifying agent commercially available from Givaudan Roche.
BIOSIL BASICS SPQ: silicone quaternium-13 slip/conditioning agent commercially available from Biosil Technologies, Inc.
BRIJ 72: steareth 2 emulsifier commercially available from Uniqema.
BRIJ 721: steareth 20 emulsifier commercially available from Uniqema.
CETIOL SN DEO: cetearyl isononanoate emollient commercially available from Sidobre Sinnova.
CARBOPOL EDT 2020: acrylic acid C10/C30 alkyl acrylate crosspolymer commercially available from BF Goodrich.
CRODESTA SL-40: sucrose cocate skin conditioning agent commercially available from Croda Oleochemicals.
DIMETHICONE 47V-100: dimethicone 100 centistokes emollient commercially available form Rhodia.
DUB LIQUIDE: 90% cetearyl octanoate/10% isopropyl myristate emollient.
FINSOLV TN: C₁₂-C₁₅ alkyl benzoate solubilizing agent commercially available from Finetex.
FUCOGEL 1000R: 2% aqueous solution of biosaccharide gum-1 skin conditioning agent commercially available from Solabia.
GLYCEROX 767: PEG-6 capric/caprylic glycerides commercially available from Croda.
GLYPURE: 70% aqueous solution of glycolic acid commercially available from Clariant
LANETTE 16: cetyl alcohol emollient/emulsifier commercially available from Sidobre Sinnova.
LAMEFORM TGI FL: polyglyceryl-3-diisostearate emulsifying agent commercially available from Sidobre Sinnova.
LUBRAJEL: humectant comprising a mixture of 32% water; 67% glyceryl polymethacrylate, 67% propylene glycol commercially available from Black.
MIRASIL CM5: cyclomethicone skin conditioning agent commercially available from SACI
MIRASIL DM 100: dimethicone skin conditioning agent commercially available form SACI
ORGANOSAL 2002D NATCOS: nylon 12 matifying agent commercially available from Atochem
PEMULEN TR1: acrylates/10-30 alkyl acrylate crosspolymer commercially available from BFGoodrich.
PHENONIP: mixture of phenoxyethanol, methylparaben, ethylparaben, propylparaben, and butylparaben commercially available from Nipa Laboratories, Inc
POLYSYNLANE: hydrogenated polyisobutene skin conditioning agent commercially available from Rossow.
PRICERINE 9091: glycerine humectant commercially available form Uniqema
SP-10: nylon-12 commercially available from Kobo Products.
STABILIEZE QM: PVM/MA decadiene crosspolymer commercially available from ISP Technologies.
WICKENOL 171: octyl hydroxystearate emollient commercially available from Alzo Inc.
WINDSOR TALC 66: talc commercially available from Luzenac/Royston.

### EXAMPLE 1

The following formula was made in accordance with the teachings of this invention. Deionized water and sodium hydroxide were added to a kettle followed by Lubragel with continuous mixing. The mixture was heated to 50°C with stirring. Then, L-tyrosine was added and the mixture was heated to 80-85°C. In a separate container DUB LIQUIDE, cetyl palmitate, LANETTE 16, MIRACIL CM5, LAMEFORM TGI FL, tocopheryl acetate, phenoxyethanol, methylparaben, propylparaben were mixed and heated until homogeneous. Then AMPHISOL A was added and the whole mixture was heated to 90°C. When, both kettles were at the desired temperatures, oil phase was added to the water phase at 80-85°C with mixing. CETIOL SN DEO and CARBOPOL EDT 2020 were premixed and added to the main kettle. The heat was discontinued and the kettle was kept under stirring while cooling. At 30°C or below the DMAE/glycolic acid/citric acid/water premix was added to the kettle. The pH of the product was adjusted followed by the Perfume IFF FBD 6162 addition. The specific ingredients and weight percentages thereof are tabulated below.

| **INGREDIENT:** | **WEIGHT PERCENT:** |
|---|---|
| Deionized water | 53.9 |
| Sodium hydroxide | 0.20 |
| LUBRAJEL | 5.00 |
| L-tyrosine | 0.50 |
| DUB LIQUIDE | 8.00 |
| Phenoxyethanol | 0.50 |
| Methylparaben | 0.20 |
| Propylparaben | 0.06 |
| Cetyl palmitate | 4.00 |
| LANETTE 16 | 1.50 |
| MIRASIL CM5 | 0.5 |
| LAMEFORM TGI FL | 1.00 |
| Tocopheryl acetate | 1.00 |
| AMPHISOL A | 1.88 |
| CETIOL SN DEO | 6.0 |
| CARBOPOL EDT 2020 | 0.50 |
| Perfume IFF FBD 6162 | 0.60 |
| ***DMAE Premix:*** | |
| Deionized water | 9.0 |
| DMAE | 3.0 |
| ***Buffer Premix:*** | |
| Glycolic acid (70%)/water (30%) | 2.19 |
| Citric acid | 0.51 |

### EXAMPLE 2

The following formula was made in accordance with the teachings of this invention. Deionized water and sodium hydroxide were mixed in the main kettle. When homogeneous, PRICERINE 9091 and Lubragel were added. The mixture was heated to 50°C. Then, ORGANOSAL 2002D NATCOS and L-tyrosine were added and the mixture was kept under stirring for 15 minutes. In a separate container CARBOPOL EOT 2020, AMIGEL, POLYSYNLANE and MIRASIL CM5 were mixed and then added to the main kettle. When the mixture in the kettle was homogeneous, butylene glycol, phenoxyethanol, methylparaben and propylparaben were added. The heating was discontinued, the mixture was mixed for 15 minutes and then, FUCOGEL 1000R was added. The kettle was cooled to 30°C or below and the pre-mix of DMAE, water, citric acid and glycolic acid was added. The pH of the product was adjusted and at the end the fragrance was added. The specific ingredients and weight percentages thereof are tabulated below.

| **INGREDIENT:** | **WEIGHT PERCENT:** |
|---|---|
| Deionized water | 65.62 |
| Sodium hydroxide | 0.10 |
| L-tyrosine | 3.00 |
| ORGANOSAL 2002D NATCOS | 0.50 |
| PRICERINE 9091 | 2.00 |
| LUBRAJEL | 3.00 |
| CARBOPOL ETD 2020 | 0.50 |
| AMIGEL | 0.80 |
| POLYSYNLANE | 2.00 |
| MIRASIL CM5 | 4.00 |
| Butylene glycol | 4.00 |
| Phenoxyethanol | 0.37 |
| Methylparaben | 0.10 |
| Propylparaben | 0.03 |
| FUCOGEL 1000R | 2.00 |
| Perfume IFF FBD 6162 | 0.20 |
| ***DMAE Premix:*** | |
| Deionized water | 10.0 |
| DMAE | 1.0 |
| ***Buffer Premix:*** | |
| Glycolic acid (70%)/water (30%) | 0.46 |
| Citric acid | 0.32 |

### Example 3

The following formula was made in accordance with the teachings of this invention. In the main kettle Deionized water and sodium hydroxide were mixed when homogeneous, PRICERINE 9091 and LUBRAJEL were added. The mixture was heated to 50°C, then, ORGANOSAL 2002D NATCOS and L-Tyrosine were added. In a separate container CARBOPOL EDT 2020, AMIGEL and MIRASIL CM5 were pre-mixed and added to the batch. When the mixture in the kettle was homogeneous, Butylene Glycol, Phenoxyethanol, Methylparaben and Propylparaben were added to it. The heating was discontinued and the mixing was kept for 15 minutes, then, FUCOGEL 1000R was added. At 30°C or below the pre-mix of DMAE, water, citric acid and glycolic acid was added to the batch. The pH of the batch was adjusted and the Perfume IFF FBD 6162 was added. The formulation had an initial pH of 6.7 at 25°C. After 24 hours the pH remained 6.7 at 25°C. The specific ingredients and weight percentages thereof are tabulated below.

| **INGREDIENT:** | **WEIGHT PERCENT:** |
|---|---|
| Deionized water | 66.0 |
| Sodium hydroxide | 0.10 |
| L-tyrosine | 0.50 |
| ORGANOSAL 2002D NATCOS | 0.25 |
| PRICERINE 9091 | 2.00 |
| LUBRAJEL | 3.00 |
| CARBOPOL ETD 2020 | 0.50 |
| AMIGEL | 0.80 |
| MIRASIL CM5 | 4.00 |
| Butylene glycol | 4.00 |
| Phenoxyethanol | 0.37 |
| Methylparaben | 0.10 |
| Propylparaben | 0.03 |
| FUCOGEL 1000R | 2.00 |
| Perfume IFF FBD 6162 | 0.20 |
| ***DMAE Premix:*** | |
| Deionized water | 10.0 |
| DMAE | 3.0 |
| ***Buffer Premix:*** | |
| Glycolic acid (70%)/water (30%) | 1.6 |
| Citric acid | 1.20 |

### EXAMPLE 4

The following formula was made in accordance with the teachings of this invention. Deionized water was added to a kettle and heated to about 78 to about 80°C. At about 78 to about 80°C, STABILEZE QM was added using a propeller mixer. The mixture was held at about 78 to about 80°C until clear. Heating was discontinued and when the mixture was at about 75°C, disodium EDTA, CRODESTA SL-40, GLYCEROX 767, and hexylene glycol were added. At about 40°C, the tyrosine/DMAE premix was added to the mixture and mixed well. The DMAE/tyrosine premix was prepared as follows: deionized water, DMAE, and tyrosine were added to a closed container and placed in a heated (50-50°C) water bath. The mixture was heated to about 50 to about 55°C. The mixture was held at that temperature with mixing until the tyrosine dissolved.

The pH of the mixture was adjusted to about 7.0 to about 7.5 with the glycolic/citric buffer premix. The remaining ingredients were added with mixing in the following order: SP-10, talc, BIOSIL BASICS SPQ, ethanol, PHENONIP, and deionized water. The mixture was homogenized at 40% for about 3-4 minutes with a rotor-stator homogenizer.

| **INGREDIENT:** | **WEIGHT PERCENT:** |
|---|---|
| Deionized water | 54.06 |
| STABILEZE QM | 1.50 |
| Disodium EDTA | 0.10 |
| CRODESTA SL-40 | 0.75 |
| GLYCEROX 767 | 0.75 |
| Hexylene glycol | 1.00 |
| ***DMAE*/*Tyrosine Premix:*** | |
| DMAE | 3.00 |
| Deionized water | 30.00 |
| L-tyrosine | 0.50 |
| ***Buffer Premix:*** | |
| GLYPURE | 2.30 |
| Citric acid | 0.80 |
| Delonized water | 1.24 |
| ***Post Addition:*** | |
| WINDSOR Talc 66 | 0.50 |
| SP-10 | 1.00 |
| BIOSIL Basics SPQ | 1.00 |
| Ethanol | 0.50 |
| Phenonip | 1.00 |

### EXAMPLE 5

The following formula was made in accordance with the teachings of this invention. Deionized water was added to a kettle and heated to about 78 to about 82°C. At about 78 to about 82°C, STABILEZE QM was added using a propeller mixer. The mixture was held at about 78 to about 80°C until clear. Heating was discontinued and the mixture was cooled to about 75°C, disodium EDTA, CRODESTA SL-40, GLYCEROX 767, and hexylene glycol were added. At about room temperature, the DMAE premix was added to the mixture and mixed well. The tyrosine premix was prepared as follows: deionized water and urea were added to a closed container and mixed. After the urea was solubilized, L-tyrosine was added to the mixture with mixing. The mixture was mixed until added to the formulation.

The pH of the mixture was adjusted to about 6.5 to about 7.0 with the glycolic/citric buffer premix. The remaining ingredients were added with mixing in the following order: SP-10, talc, BIOSIL BASICS SPQ, ethanol, PHENONIP, and deionized water. The mixture was homogenized at 40% for about 2 minutes with a rotor-stator homogenizer.

| **INGREDIENT:** | **WEIGHT PERCENT:** |
|---|---|
| Deionized water | 62.59 |
| STABILEZE QM | 1.50 |
| Disodium EDTA | 0.10 |
| CRODESTA SL-40 | 0.75 |
| GLYCEROX 767 | 0.75 |
| Hexylene glycol | 1.00 |
| ***DMAE Premix:*** | |
| DMAE | 3.00 |
| Deionized water | 3.00 |
| ***Tyrosine*/*Urea Premix:*** | |
| Deionized water | 10.00 |
| Urea | 5.00 |
| L-tyrosine | 5.00 |
| ***Buffer Premix:*** | |
| GLYPURE 70 | 2.61 |
| Citric acid | 0.70 |
| ***Post Addition:*** | |
| WINDSOR Talc 66 | 0.50 |
| SP-10 | 1.00 |
| BIOSIL Basics SPQ | 1.00 |
| Ethanol | 0.50 |
| PHENONIP | 1.00 |

### EXAMPLE 6

The following formula was made in accordance with the following procedure. Deionized water was added to a kettle and heated to about 78 to about 80°C. At about 78 to about 80°C, STABILEZE QM was added using a propeller mixer. The mixture was held at about 78 to about 80°C until uniform. Heating was discontinued and when the mixture was at about 75°C, disodium EDTA, CRODESTA SL-40, GLYCEROX 767, and hexylene glycol were added. At about room temperature, the tyrosine/DMAE premix was added to the mixture and mixed well. The DMAE/tyrosine premix was prepared as follows: deionized water, DMAE and tyrosine were added to a closed container and placed in a heated (50-55°C) water bath. The mixture was heated to about 50 to about 55°C. The mixture was held at that temperature with mixing until the tyrosine dissolved.

The pH of the mixture was adjusted to about 7.0 to about 7.5 with the glycolic/malic buffer premix. The remaining ingredients were added with mixing in the following order: SP-10 talc, BIOSIL BASICS SPQ, ethanol, PHENONIP, and deionized water. The mixture was homogenized at 40% for about 3-4 minutes with a rotor-stator homogenizer. The specific ingredients and proportions thereof are tabulated below.

| **INGREDIENT:** | **WEIGHT PERCENT:** |
|---|---|
| Deionized water | 55.16 |
| STABILEZE QM | 1.50 |
| Disodium EDTA | 0.10 |
| CRODESTA SL-40 | 0.75 |
| Glycerox 767 | 0.75 |
| Hexylene glycol | 1.00 |
| ***DMAE*/*Tyrosine Premix:*** | |
| DMAE | 3.00 |
| Deionized water | 30.00 |
| L-tyrosine | 0.50 |
| ***Buffer Premix:*** | |
| Glypure | 1.20 |
| Malic acid | 0.80 |
| Deionized water | 1.24 |
| ***Post Addition:*** | |
| Winnsor Talc 66 | 0.50 |
| SP-10 | 1.00 |
| BIOSIL Basics SPQ | 1.00 |
| Ethanol | 0.50 |
| Phenonip | 1.00 |

### EXAMPLE 7

The following formula was made according to the following procedure: Deionized water was added to a kettle and heated to about 78 to about 80°C. At about 78 to about 82°C, STABILEZE QM was added using a propeller mixer. The mixture was held at about 78 to about 82°C until clear. Heating was discontinued and when the mixture was at about 75°C, disodium EDTA, CRODESTA SL-40, GLYCEROX 767, and hexylene glycol were added. At room temperature, the DMAE premix was added to the mixture to neutralize the STABILEZE followed by the tyrosine premix and mixed well. The pH of the mixture was adjusted to about 6.5 to about 7.0 with glycolic acid. The remaining ingredients were added with mixing in the following order: SD-10, talc, BIOSIL BASICS SPQ, ethanol, PHENONIP, and deionized water. The mixture was homogenized at 40% for about 2 minutes with a rotor-stator homogenizer.

| **INGREDIENT:** | **WEIGHT PERCENT:** |
|---|---|
| Deionized water | 63.3 |
| STABILEZE QM | 1.50 |
| Disodium EDTA | 0.10 |
| CRODESTA SL-40 | 0.75 |
| GLYCEROX 767 | 0.75 |
| Hexylene glycol | 1.00 |
| ***DMAE Premix:*** | |
| DMAE | 3.00 |
| Deionized water | 3.00 |
| ***Tyrosine*/*Urea Premix:*** | |
| Deionized water | 10.00 |
| Urea | 5.00 |
| L-tyrosine | 5.00 |
| ***Post Addition:*** | |
| GLYPURE | 2.61 |
| Windsor Talc 66 | 0.50 |
| SP-10 | 1.00 |
| BIOSIL Basics SPQ | 1.00 |
| Ethanol | 0.50 |
| PHENONIP | 1.00 |

### EXAMPLE 8

Examples 1 and 2 were evaluated on panels of 140 consumers in a four-week test. As show by the table below, consumers recognized improvements in skin firmness, less sagging, tightening of the skin, improved facial shape and contours.

| **Benefits** | **Example 1** | **Example 2** |
|---|---|---|
| Helps skin look firmer under eyes | 66* | 69 |
| Helps your skin look firmer | 66 | 71 |
| Visibly improves skin appearance | 70 | 74 |
| Firms your skin all day | 62 | 72 |
| Defines and reshapes the contours of your face | 43 | 45 |
| Visibly reduces the appearance of sagging skin | 50 | 60 |
| Lifts your facial skin | 51 | 58 |
| Visibly re-tightens your skin | 57 | 65 |

| | | |
|---|---|---|
| *Percentage of consumers agreeing with statement | | |

### EXAMPLE 9

This example illustrates the improved pH stability of compositions containing a combination of citric and glycolic acid.

| Example | Buffer Type | pH drop |
|---|---|---|
| Example 5 | Glycolic/citric acid | 0.1 |
| Example 6 | Glycolic/malic | 0.4 |
| Example 8 | Glycolic acid | 0.4 |

Having described the invention with reference to particular compositions, theories of effectiveness, and the like, it will be apparent to those of skill in the art that it is not intended that the invention be limited by such illustrative embodiments or mechanisms, and that modifications can be made without departing from the scope or spirit of the invention, as defined by the appended claims. The claims are meant to cover the claimed components and steps in any sequence which is effective to meet the objectives there intended, unless the context specifically indicates the contrary.

## Claims

1. A composition for use in improving skin firmness, in improving the appearance of facial contours or in reducing the appearance of sagging skin by topical application comprising:
(a) an effective amount of a compound of the formula: 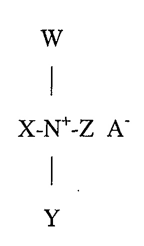 wherein W, X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group, and wherein A is a mixture of anionic counterions derived from at least two pharmaceutically acceptable acids and esters thereof; and
(b) a cosmetically acceptable carrier.

2. The composition of claim 1, wherein at least one of said acids is an alpha hydroxy acid.

3. The composition of claim 1 or claim 2, wherein said acids are selected from glycolic acid, citric acid, malic acid, and mixtures thereof.

4. The composition of any one of claims 1 to 3, wherein said anionic counterion is derived from a mixture of glycolic acid and citric acid.

5. The composition of claim 4, wherein the ratio of citric acid to glycolic acid ranges from 1:1 to 1:5.

6. The composition of any one of claims 1 to 3, wherein said anionic counterion is derived from a mixture of glycolic acid and malic acid.

7. The composition of claim 6, wherein the ratio of malic acid to glycolic acid ranges from 1:1 to 1:5.

8. The composition of any one of claims 1 to 7, wherein the pH of said composition ranges from 4.5 to 8.5.

9. The composition of any one of claims 1 to 8, wherein said compound is present at about 10% by weight.

10. The composition of any one of claims 1 to 9, wherein said composition is incorporated into a material carrier selected from a dry wipe, a wet wipe, a puff, a hydrogel matrix, an adhesive patch, or a non-adhesive patch.
